# EUROPÄISCHE PATENTANMELDUNG

(11) **EP 2 530 463 A1**
(43) Veröffentlichungstag der Anmeldung: **05.12.2012**
(21) Anmeldenummer: 11164137.9
(22) Anmeldetag: 28.04.2011
(51) Int. Cl.: G01N 33/52, A61B 5/103, A61K 49/00

(54) **Indikator zur Ermittlung des Hautzustands**

(30) Priorität: 19.01.2011 DE 102011008899
(71) Anmelder: USP Indicator Solutions GmbH, 9020 Klagenfurt (AT)
(72) Erfinder: Lengfeldner, Erhard, 9020 Klagenfurt (AT)
(74) Vertreter: Preissner, Nicolaus

(57) **Zusammenfassung**

Ein Indikator (22) zur Ermittlung des Hautzustands weist einen Träger (26) und eine auf dem Träger (26) aufgebrachte Schicht einer grafischen Druckfarbe (34) auf, wobei die Druckfarbe (34) eine Bindemittelmatrix (36) aufweist und wobei die Bindemittelmatrix (36) wenigstens ein darin homogen dispergiertes Additiv (46) enthält, das zur irreversiblen Veränderung der optischen Eigenschaften der Bindemittelmatrix (36) bei Kontakt mir Hautsekret ausgebildet ist.

## Beschreibung

Die vorliegende Erfindung betrifft einen Indikator zur Ermittlung des Hautzustands sowie ein Verfahren zur Herstellung eines solchen Indikators.

Die Haut als lebendes Organ kann ihre physikalischen und chemischen Eigenschaften laufend ändern und sich so den Einflüssen der Umgebung anpassen. Diese Reaktionen wurden in früheren Zeiten kaum beachtet, was sich besonders in einer recht starren Hautpflegeberatung niederschlug. Durch genauere Befunde ist es möglich, Hautpflegepläne dynamischer zu entwickeln und so die Gesundheit der Haut zu optimieren.

Mit aktuellem Hautzustand bezeichnet man denjenigen Hautzustand, der sich am Untersuchungszeitpunkt bietet. Je nach Fett- und Feuchtigkeitsgehalt der Haut kann zu unterschiedlichen Untersuchungszeitpunkten ein unterschiedlicher Hautzustand vorliegen. Der aktuelle Hautzustand ist abhängig von:
- Vererbung (zum Beispiel Anlage zur Sebostase, Seborrhö oder Akne)
- Inneren Faktoren (zum Beispiel hormonelle Störungen, Ovulationshemmer, Polyzystische Ovarien, Stress oder Rauchen),
- Äußeren Faktoren (Wärme, Kälte, Meerwasser, entfettende Seifen, Lösungsmittel).

Der aktuelle Hautzustand kann wie folgt eingeteilt werden:
1. Normalhaut
2. fett-feuchte Haut
3. fett-trockene Haut
4. fettarm-trockene Haut
5. fettarm-feuchte Haut

Normalhaut: Dieser Hautzustand ist feinporig, zart, samtig-glatt, geschmeidig, rosig, fettet nicht nach und spannt nicht. Die Haut wirkt gesund, ist problemlos zu reinigen und zu pflegen.

Fett-feuchter Hautzustand: Typisch für diesen Hautzustand ist eine grobe, kräftige, stark glänzende, großporige Haut. Der Hautzustand ist sehr robust, widerstandsfähig und auch unempfindlich gegen Sonneneinstrahlung. Die Fett-Feuchte Haut tritt vorwiegend auf dem Kopf, an Brust und Rücken auf. Die übrige Körperhaut kann dabei normal oder sogar fettarm sein. Da sich mit Beginn der Pubertät dieser Hautzustand entwickelt, neigt ein hoher Anteil der Jugendlichen zu diesem Hautzustand.

Fett-trockener Hautzustand: Dieser Zustand tritt als kurzzeitige Abweichung des Fett-Feuchten Hautzustands auf. Dabei hat die Haut einen glänzenden Schimmer, eventuell eine starke Schuppung, ein pergamentartiges Erscheinungsbild und ein unangenehmes Spannungsgefühl. Nach extremer Sonnenbestrahlung (Urlaub, Sonnenbrand, Solarium) oder intensiver externer Aknetherapie kann dieser Zustand eintreten.

Fettarm-trockener Hautzustand: Die Talgdrüsen produzieren zu wenig Talg, die Haut erscheint schuppig, rau, matt und glanzlos. Es können Spannungen, Juckreiz und Ekzeme entstehen. Die oft fleckförmigen Rötungen und trockenen, rauen Stellen geben der Haut ein unruhiges Erscheinungsbild. Durch den verminderten Fett- und Feuchtigkeitsgehalt besteht eine Tendenz zur vorzeitigen Alterung der Haut, das heißt es können Fältchen und Runzeln auftreten. Die Hautschichten verdünnen sich.

Fettarm-feuchter Hautzustand: Dem nur kurzzeitig auftretenden Fettarm-Feuchten Hautzustand liegt die Fettarm-Trockene Haut zugrunde. Durch äußere Umstände, zum Beispiel Schwitzen beim Sport ändert sich dieser Hautzustand. Die Patienten beschreiben eine aufflammende Rötung wie bei einer Entzündung. Außerdem entsteht ein sehr unangenehmes Hitzegefühl, das lange Zeit anhalten kann.

Die Hautpflegeberatung sollte sich immer streng am Erscheinungsbild und der Hautbeschaffenheit jedes einzelnen Patienten orientieren. Bei einem Dermatologen oder einer Kosmetikerin wird der Hautzustand daher aufwendig durch beispielsweise Kamerasysteme bestimmt. Solche Verfahren sind dem Patienten zu Hause nicht möglich.

Der Patient muss jedoch auch in die Lage versetzt werden, die Bedürfnisse seiner Haut selber besser zu verstehen und zu beurteilen. Dazu muss er in die Lage versetzt werden, nicht nur selbständig seinen Hautzustand zu untersuchen, er muss dies auch vergleichend, d.h. zu verschiedenen Zeitpunkten, durchführen können, wobei die Ergebnisse miteinander verglichen werden. Dazu sollte das Ergebnis irreversibel auch nach einer längeren Zeit beispielsweise von einem Hautzustandsindikator ablesbar sein.

Aufgabe der Erfindung ist es, einen Indikator zur Ermittlung des Hautzustands zur Verfügung zu stellen, der den aktuellen Hautzustand irreversibel anzeigt.

Diese Aufgabe wird durch einen Indikator zur Ermittlung des Hautzustands mit den Merkmalen des Anspruchs 1 gelöst. Ein Verfahren zur Herstellung eines solchen Indikators ist Gegenstand des Nebenanspruches.

Vorteilhafte Ausgestaltungen sind Gegenstand der Unteransprüche.

Ein Indikator zur Ermittlung des Hautzustands weist einen Träger und eine auf dem Träger aufgebrachte Schicht einer graphischen Druckfarbe auf. Die Druckfarbe weist eine Bindemittelmatrix auf, wobei die Bindemittelmatrix wenigstens ein darin homogen dispergiertes Additiv enthält, das zur irreversiblen Veränderung der optischen Eigenschaften der Bindemittelmatrix bei Kontakt mit Hautsekret ausgebildet ist.

Wird ein solcher Indikator von einem Patienten auf eine Hautregion aufgedrückt, bewirkt das in dieser Region vorliegende Hautsekret, das Sebum und Feuchtigkeit aufweist, dass das Additiv die optischen Eigenschaften der Bindemittelmatrix verändert. Dadurch werden auf dem Indikator Fett- und/oder Feuchtigkeit sichtbar und der Patient kann durch die Menge der Veränderung in der Bindemittelmatrix bestimmen, wie viel Feuchtigkeit beziehungsweise Sebum die Haut in dieser Region absondert und somit beurteilen, welcher Hautzustand in der untersuchten Hautregion vorliegt. Es ist ihm nach diesem Test möglich, ein entsprechendes Pflegeprodukt zu verwenden. Außerdem zeigt der Indikator durch die Irreversibilität der optischen Veränderung auch nach längerer Zeit noch den Hautzustand an, sodass eine Entwicklung des Hautzustandes und damit eine Wirkung eines Pflegeproduktes verfolgt werden kann.

Das wenigstens eine Additiv ist vorzugsweise zur irreversiblen Veränderung der optischen Eigenschaften der Bindemittelmatrix bei Kontakt mit Feuchtigkeit ausgebildet. Nach Applikation des Indikators auf einer Hautregion ist es dem Patienten somit möglich festzustellen, ob diese Hautregion eine hohe oder niedrige Feuchtigkeit aufweist und dementsprechend feuchtigkeitsspendende Pflegeprodukte benötigt.

Vorzugsweise ist das wenigstens eine Additiv bei Kontakt mit Feuchtigkeit zum irreversiblen Einfärben der Bindemittelmatrix ausgebildet. Die Farbgebung der Bindemittelmatrix unterscheidet sich somit vorzugsweise nach Kontakt mit Feuchtigkeit von der Farbgebung vor dem Feuchtigkeitskontakt. Durch die Farbintensität und Menge der verfärbten Bereiche ist zu erkennen, ob die Hautregion viel Feuchtigkeit oder wenig Feuchtigkeit aufweist.

Vorteilhaft weist das wenigstens eine Additiv einen wasserlöslichen Farbstoff auf, der Partikel mit einer Größe von 1 bis 5 µm aufweist, wobei die Partikel bei Reaktion mit Feuchtigkeit zum Erzeugen einer Farbgebung in der Bindemittelmatrix zum Expandieren und Ausbluten in die Bindemittelmatrix ausgebildet sind. Die Farbstoffpartikel liegen vorteilhaft als freie Festkörper fein verteilt in der Bindemittelmatrix vor. Durch die geringe Partikelgröße sowie die feine Verteilung in der Bindemittelmatrix ist die Farbgebung des wasserlöslichen Farbstoffes zunächst nicht zu sehen. Erst nach Kontakt mit Feuchtigkeit beginnt sich der Farbstoff in der Feuchtigkeit, zu lösen und bläht auf. Der gelöste Farbstoff diffundiert vollständig in die Bindemittelmatrix, das heißt er blutet aus. Die Bindemittelmatrix wird so durch den Farbstoff eingefärbt. Auch nachdem der Indikator abgetrocknet ist, ist die Bindemittelmatrix weiterhin mit der Farbgebung des Farbstoffes eingefärbt. Eine Rückbildung zu kleinen, fein dispergierten Partikeln ist nicht mehr möglich, so dass die Farbgebung irreversibel in der Bindemittelmatrix erhalten bleibt. Die Bestimmung des Feuchtigkeitsgehaltes der Haut ist somit vorteilhaft auch über einen längeren Zeitraum gewährleistet.

Vorzugsweise ist der wasserlösliche Farbstoff ein Lebensmittelfarbstoff und/oder die Bindemittelmatrix ist wasserfrei und/oder die Bindemittelmatrix ist ölfrei und/oder die Bindemittelmatrix ist porös. Lebensmittelfarbstoffe haben den Vorteil, dass sie bezüglich der Verträglichkeit geprüft und zugelassen sind. Eine Applikation auf der Haut trägt somit keine allergischen Reaktionen mit sich und ist daher unproblematisch. Es ist von Vorteil, wenn die Bindemittelmatrix wasserfrei ist, um zu vermeiden, dass der wasserlösliche Farbstoff bereits ohne Kontakt mit der Haut in die Bindemittelmatrix ausblutet und diese einfärbt.

In besonders bevorzugter Ausgestaltung weist die Bindemittelmatrix ein zweites darin homogen dispergiertes Additiv auf, das zum irreversiblen Verändern der optischen Eigenschaften der Bindemittelmatrix bei Kontakt mit Sebum ausgebildet ist. Dabei ist das zweite Additiv bei Kontakt mit Sebum vorzugsweise zum Verändern der Transmissionseigenschaften der Bindemittelmatrix für sichtbares Licht ausgebildet. Die Bindemittelmatrix ist vorzugsweise ölfrei und/oder porös. Durch die Porösität der Bindemittelmatrix wird auftreffendes Licht vorteilhaft gestreut, so dass der Indikator undurchsichtig erscheint. Wird über eine der Poren Sebum aufgenommen, verändern sich an dieser Stelle des Indikators die strukturellen Eigenschaften, wobei das auftreffende Licht kann nicht mehr gestreut werden kann, so dass der Hintergrund des Indikators an dieser Stelle durchscheinen kann. Der Indikator, insbesondere die Bindemittelmatrix, wird somit für sichtbares Licht durchsichtig.

Da die Bindemittelmatrix an Stellen, die in Kontakt mit Sebum waren, durchsichtig wird und sich die Bindemittelmatrix an Stellen, die in Kontakt mit Feuchtigkeit waren, verfärbt, kann sowohl der Sebumgehalt als auch der Feuchtigkeitsgehalt der getesteten Hautregion bestimmt werden.

Vorzugsweise weist das zweite Additiv wenigstens eine der folgenden Eigenschaften auf:
- es ist aus einer Gruppe ausgewählt, die SiO₂ Al₂O₃, Ca₃PO₄, Talkum und (Ca₅(PO₄)₃ * OH) umfasst;
- es ist Kristallin und liegt in einem Größenbereich von 1 bis 30 µm oder als Partikel in einem Größenbereich von unter 1 µm oder als Agglomerat in einem Größenbereich bis 300 µm vor.

In bevorzugter Ausgestaltung weist die Druckfarbe eine wasserfreie erste Bindemittelmatrix mit darin dispergiertem ersten Additiv und eine ölfreie zweite Bindemittelmatrix mit darin dispergiertem zweiten Additiv auf. Somit ist es vorteilhaft möglich, mit einem Indikator sowohl den Sebumgehalt als auch den Feuchtigkeitsgehalt der Haut zu bestimmen, ohne dass das reaktive Additiv bereits vor Kontakt mit der Haut die optischen Eigenschaften der Bindemittelmatrizen verändert.

Bevorzugt ist die Druckfarbe eine Offset-Druckfarbe, eine Siebdruckfarbe oder eine Digitaldruckfarbe, insbesondere eine Inkjet-Druckfarbe. Weiter bevorzugt ist der Träger aus Papier oder Kunststoff gebildet. Der Indikator kann so vorzugsweise in einem gewöhnlichen Druckereibetrieb günstig und einfach hergestellt werden.

Vorteilhaft weist der Träger eine Farbgebung auf, die unterschiedlich zu einer Farbgebung der Bindemittelmatrix vor Kontakt des zweiten Additivs mit Sebum ist und die unterschiedlich zu der Farbgebung der Bindemittelmatrix nach Kontakt des ersten Additivs mit Feuchtigkeit ist. Die Farbgebung des Trägers scheint so nach Kontakt des Indikators mit Sebum durch die durchsichtig gewordene Bindemittelmatrix durch. Sie unterscheidet sich jedoch gleichzeitig von der Farbe der eingefärbten Bindemittelmatrix nach dem Kontakt mit Feuchtigkeit, so dass durch die verschiedene Farbgebung einfach unterschieden werden kann, welche Mengen an Sebum beziehungsweise Feuchtigkeit in der getesteten Hautregion vorliegen.

Vorteilhaft kann ist benachbart zu der Schicht der graphischen Druckfarbe auf dem Träger ein Vergleichsmaßstab zur Anzeige des Hautzustands angeordnet sein. Der Maßstab zeigt vorteilhaft die mengenmäßige Verteilung der unterschiedlichen Farbgebung je nach Hautzustand an, so dass es dem Patienten einfach möglich ist, durch Vergleich zu erkennen, zu welchem Hautzustand er die getesteten Hautregion zuordnen soll.

Vorteilhaft weist der Träger eine erste Schicht einer ersten Druckfarbe mit einer wasserfreien Bindemittelmatrix und einem darin dispergierten ersten Additiv auf und eine zweite Schicht einer zweiten Druckfarbe mit einer ölfreien Bindemittelmatrix und einem darin dispergierten zweiten Additiv. Die Schichten können beispielsweise graphisch nebeneinander aufgedruckt werden oder sich ringförmig umschließen. Im Falle eines Hauttestes zeigt dann die erste Schicht lediglich den Feuchtigkeitsgehalt der Haut an, während die zweite Schicht den Sebumgehalt der Haut aufzeigt. Farbüberlagerungen können so vorteilhaft vermieden werden.

In einem Verfahren zur Herstellung eines Indikators zur Anzeige des Hautzustands wird zunächst ein Additiv in einer Bindemittelmatrix dispergiert, dann diese Bindemittelmatrix mit einer graphischen Druckfarbe vermischt, ein Träger bereitgestellt, und dann wenigstens eine Schicht der Druckfarbe mittels herkömmlicher Drucktechnik, beispielsweise Siebdruck, auf den Träger aufgebracht.

Vorzugsweise wird beim Dispergieren des Additivs zunächst ein wasserlöslicher Farbstoff zu einer Partikelgröße von 1 bis 5 µm zermahlen und eine wasserfreie und/oder ölfreie Bindemittelmatrix gewählt.

Weiter vorteilhaft wird ein zweites Additiv in einer zweiten Bindemittelmatrix dispergiert, diese zweite Bindemittelmatrix mit einer graphischen zweiten Druckfarbe vermischt und eine zweite Schicht der zweiten Druckfarbe mittels herkömmlicher Drucktechnik auf den Träger aufgebracht.

Vorzugsweise wird die erste beziehungsweise die zweite Druckfarbe mittels Siebdruck, Tiefdruck oder Flexidruck auf den Träger aufgebracht.

Besonders bevorzugt wird die erste und/oder die zweite Schicht mit einer Trockenschichtdicke von 15 bis 20 µm aufgebracht. Weiter vorteilhaft wird die erste und/oder die zweite Druckfarbe in einer graphischen Gestaltung auf den Träger aufgebracht.

In besonders bevorzugter Ausgestaltung wird auf den Träger benachbart zu der ersten und/oder der zweiten Schicht ein Vergleichsmaßstab aufgebracht.

Ausführungsbeispiele der Erfindung werden nachfolgend anhand der beigefügten schematischen Zeichnungen erläutert. Darin zeigt:
- Fig. 1: eine Gesichtsdarstellung mit mehreren unterschiedlichen Hautzustandsregionen;
- Fig. 2: mehrere gestalterische Ausführungsformen eines Indikators zur Ermittlung des Hautzustands;
- Fig. 3: eine erste Ausführungsform des Indikators zur Ermittlung des Hautzustands;
- Fig. 4: eine zweite Ausführungsform des Indikators zur Ermittlung des Hautzustands;
- Fig. 5: Vergleichsmaßstäbe der Hautzustandindikatoren aus Fig. 3 und Fig. 4;
- Fig. 6: die Lichtbrechung des Hautzustandindikators nach Fig. 2 oder Fig. 3 vor und nach Kontakt mit Sebum;
- Fig. 7: die Farbverhältnisse des Hautzustandindikators nach Fig. 2 oder Fig. 3 vor und nach Kontakt mit Feuchtigkeit;
- Fig. 8: eine erste Kombination der Effekte aus Fig. 6 und 7;
- Fig. 9: eine zweite Kombination der Effekte aus Fig. 6 und 7;
- Fig. 10: die Verwendung eines Hautzustandsindikators;
- Fig. 11: verschiedene Ausführungsformen des Hautzustandsindikators mit Farbgebung nach Verwendung bei fett-trockener Haut;
- Fig. 12: verschiedene Ausführungsformen des Hautzustandsindikators mit Farbgebung nach Verwendung bei fett-feuchter Haut;
- Fig. 13: eine erste Möglichkeit, den Hautzustandsindikator einer Zeitschrift beizulegen; und
- Fig. 14: eine zweite Möglichkeit, den Hautzustandsindikator einer Zeitschrift beizulegen.

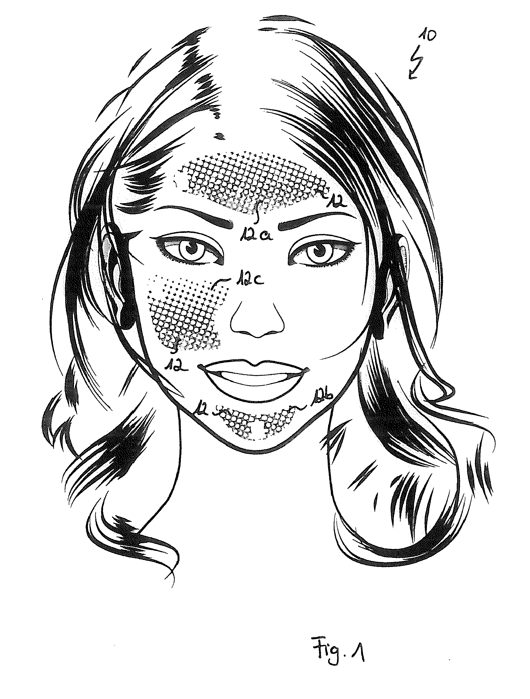

Fig. 1 zeigt eine Gesichtsdarstellung 10 mit unterschiedlichen Hautzustandregionen 12. In einer Stirnregion 12a und einer Kinnregion 12b herrschen zumeist fett-feuchte Verhältnisse mit einer großen Absonderung von Sebum durch die dort lokalisierten Talgdrüsen. In Wangenregionen 12c ist die Haut dagegen zumeist fettarm und trocken. Um eine optimale Pflege für die jeweiligen Hautzustandregionen 12 zu wählen, ist es vorteilhaft, wenn zuvor an den gekennzeichneten Hautzustandregionen 12 die Menge des abgesonderten Sebums beziehungsweise der abgesonderten Feuchtigkeit gemessen wird.

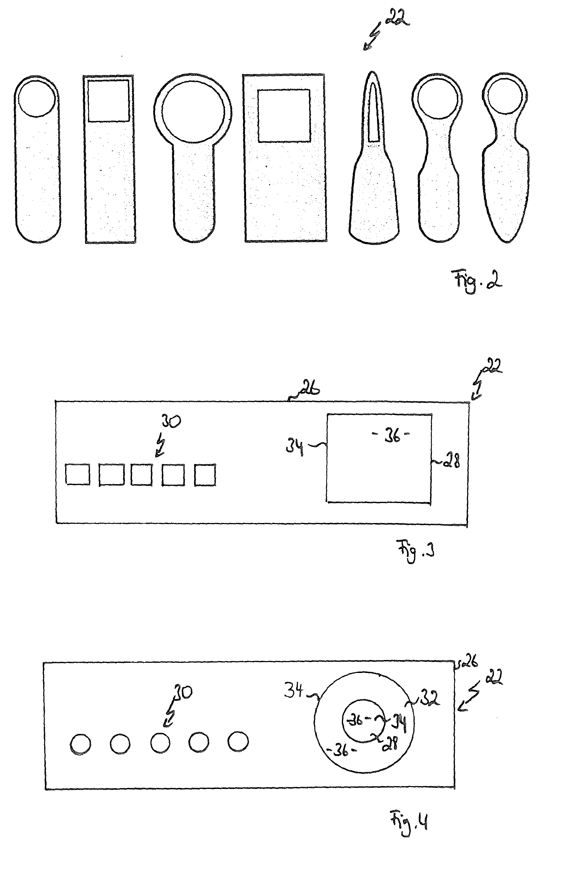

Dies kann mit einem Indikator 22 zur Bestimmung des Hautzustands - kurz: Hautzustandindikator- , durchgeführt werden. Mehrere mögliche gestalterische Ausführungsformen des Indikators 22 sind in Fig. 2 gezeigt.

Fig. 3 zeigt eine erste Ausführungsform des Hautzustandindikators 22. Auf einem Träger 26 ist im rechten Bereich eine erste Schicht 28 einer graphischen Druckfarbe aufgebracht. Im linken Bereich ist ein Vergleichsmaßstab 30 angeordnet. Wird nun die erste Schicht 28 auf eine der Hautzustandregionen 12 aus Fig. 1 aufgedrückt, verändern sich die optischen Eigenschaften der Schicht 28 derart, dass bestimmt werden kann, wie viel Feuchtigkeit, beziehungsweise wie viel Sebum, in der getesteten Hautzustandregion 12 von der Haut abgesondert wird.

Fig. 4 zeigt eine zweite Ausführungsform des Hautzustandindikators 22. Hierbei ist um die erste Schicht 28 ringförmig eine zweite Schicht 32 auf den Hautzustandindikator 22 aufgebracht. Die erste Schicht 28 wird dabei zur Bestimmung des Sebumgehaltes der getesteten Hautzustandregion 12 verwendet, während die zweite Schicht 32 den Feuchtigkeitsgehalt in dieser Hautzustandregion 12 anzeigt.

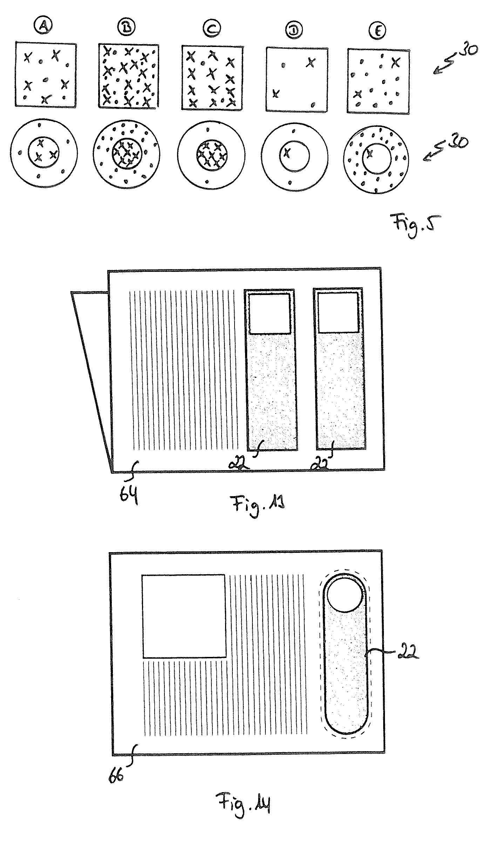

Nach Aufdrücken des Hautzustandindikators 22 kann der Hautzustand durch Vergleich mit dem Vergleichsmaßstab 30, dargestellt in Fig. 5, ermittelt werden.

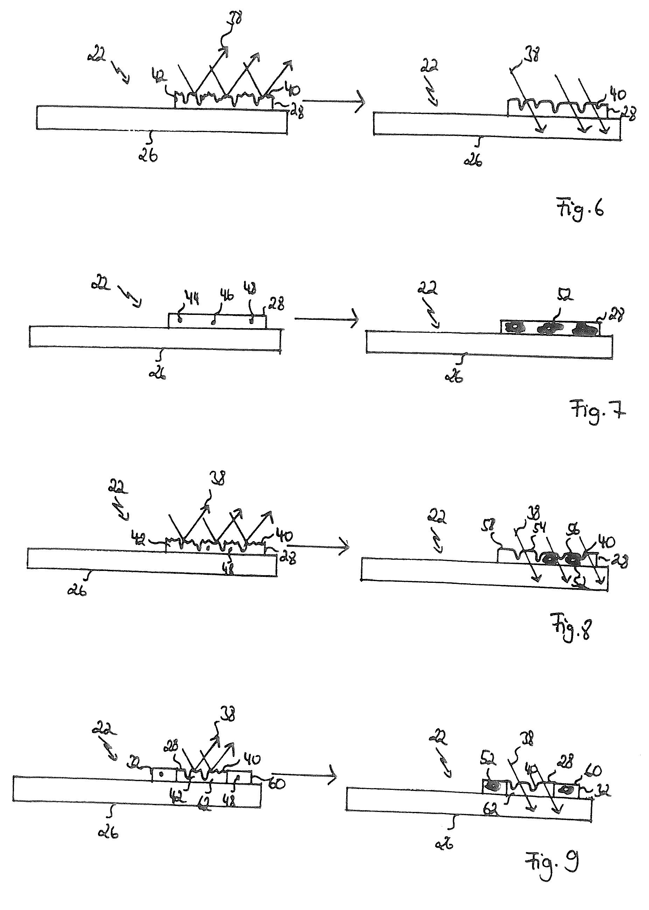

Fall A zeigt dabei den Zustand bei normaler Haut, Fall B bei fett-feuchter Haut, Fall C bei fett-trockener Haut, Fall D bei fettarm-trockener Haut und Fall E bei fettarm-feuchter Haut. Die Kreuze stehen dabei für Regionen, die mit Sebum in Kontakt gekommen sind, während die Punkte Regionen darstellen, die mit Feuchtigkeit in Kontakt gekommen sind. Der Hautzustandindikator 22 ändert an den Stellen, an denen er entweder mit Sebum oder mit Feuchtigkeit in Kontakt kommt, seine Farbe. Die Funktionsweise zur Farbänderung ist in den Fig. 6 und 7 gezeigt.

Fig. 6 zeigt die Funktionsweise des Hautzustandindikators 22 bei Kontakt mit Sebum. Eine Druckfarbe 34 wurde dabei mittels üblicher Druckverfahren auf den Träger 26 aufgebracht. Die Druckfarbe 34 weist eine poröse Bindemittelmatrix 36 mit Poren 40 auf. Einfallendes Licht 38 wird an den Kanten der Poren 40 gestreut, weswegen die Bindemittelmatrix 36 undurchsichtig erscheint. In der Bindemittelmatrix 36 ist ein Additiv 42 vorhanden, das nach Kontakt der Bindemittelmatrix 36 mit Sebum die Poren 40 verschließt und die Bindemittelmatrix 36 somit für sichtbares Licht 38 durchlässig macht. Die Farbe des Trägers 26 scheint an den Stellen der geschlossenen Poren 40 durch die Bindemittelmatrix 36 durch und zeigt so an, an welchen Stellen Sebum von dem Hautzustandindikator 22 aufgenommen worden ist. Um zu vermeiden, dass der Hautzustandindikator 22 bereits vor Kontakt mit einer Hautzustandregionen 12 reagiert, wird vorzugsweise eine ölfreie Bindemittelmatrix 36 gewählt, und die Druckfarbe 34 wird in einer ölfreien Umgebung auf den Träger 26 aufgebracht.

Fig. 7 zeigt den Hautzustandindikator 22 vor und nach Kontakt mit Feuchtigkeit. In der Bindemittelmatrix 36, die in diesem Fall vorzugsweise wasserfrei ist und in einer wasserfreien Umgebung auf den Träger 26 aufgebracht worden ist, sind fein dispergiert Partikel 44 eines Additives 46 vorhanden. Die Partikel 44 sind Partikel eines wasserlöslichen Farbstoffes 48, insbesondere eines Lebensmittelfarbstoffes 50. Die Partikel 44 sind in der Bindemittelmatrix 36 derart fein dispergiert und so klein, dass ihre Farbgebung nicht zu sehen ist. Erst bei Kontakt mit Feuchtigkeit expandieren die Partikel 44, bluten aus und geben ihre Farbgebung an die umgebende Bindemittelmatrix 36 ab. So entsteht ein Farbfleck 52, der deutlich größer ist als der ursprüngliche Partikel 44. Auch nachdem die Feuchtigkeit verdampft ist, bleibt die Farbgebung in der Bindemittelmatrix 36 erhalten, d.h. die Verfärbung der Bindemittelmatrix 36 erfolgt irreversibel. Der ermittelte Hautzustand kann somit über einen längeren Zeitraum auf dem Hautzustandsindikator 22 sichtbar.

Fig. 8 zeigt eine Kombination der Vorgänge der Fig. 6 und 7. Hier sind in der Bindemittelmatrix 36 sowohl das Additiv 42 vorhanden, das bei Kontakt mit Sebum die Bindemittelmatrix 36 durchsichtig macht, als auch das Additiv 46, dessen Partikel 44 nach Kontakt mit Feuchtigkeit in die Bindemittelmatrix 36 ausbluten und diese dabei verfärben. Hier ist es vorteilhaft, wenn der Träger 26 eine Farbgebung aufweist, die sich von der Farbgebung der Bindemittelmatrix 36 vor Kontakt mit Sebum unterscheidet, die aber auch unterschiedlich ist zu der Farbgebung der Partikel 44, die nach Kontakt mit Feuchtigkeit an die Bindemittelmatrix 36 abgegeben wird. Nachdem der Hautzustandindikator 22 auf die Haut aufgedrückt worden ist, entstehen so drei unterschiedlich eingefärbte Bereiche, ein erster Bereich 54, der die Farbgebung des Trägers 26 aufweist, ein zweiter Bereich 56, der die Farbgebung der Partikel 44 aufweist und ein dritter Bereich 58, der im Vergleich zur Farbgebung der Bindemittelmatrix 36 vor Kontakt mit der Haut keinerlei Farbveränderung aufweist. Die Farbgebung nach Kontakt mit der Haut verbleibt irreversibel auf dem Hautzustandsindikator 22.

Fig. 9 zeigt einen Hautzustandindikator 22 entsprechend der zweiten Ausführungsform nach Fig. 3. Hierbei wird in zwei unterschiedlichen Bereichen getrennt voneinander der Sebumgehalt beziehungsweise der Feuchtigkeitsgehalt der Haut bestimmt. Ein Feuchtigkeitsbereich 60 umschließt dabei ringförmig einen Sebumbereich 62. Bei Kontakt mit der Haut werden die Poren 40 im Sebumbereich 62 durch das erste Additiv 42 verschlossen und die Bindemittelmatrix 36 durchsichtig, so dass die Farbe des Trägers 26 durchscheint. Gleichzeitig bluten die Partikel 44, die in dem Feuchtigkeitsbereich 60 angeordnet sind, in die dortige Bindemittelmatrix 36 aus und bilden Farbflecke 52 an den Stellen, an denen Feuchtigkeit aufgenommen worden ist. Die Farbflecke 52 verschwinden auch nach verdampfen der Feuchtigkeit nicht, d.h. die Bindemittelmatrix 36 wurde irreversibel verfärbt.

Mit dem beschriebenen Hautzustandsindikator 22 ist es möglich, schnell und einfach, auch im Hausgebrauch, den Hautzustand an verschiedenen Stellen des Körpers irreversibel zu bestimmen und diese entsprechend mit den richtigen Produkten zu pflegen.

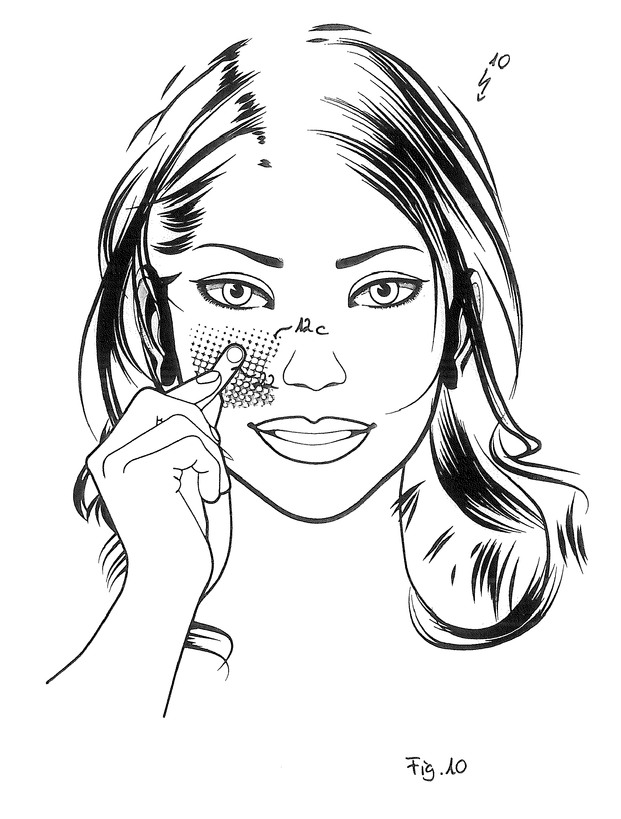

Die Verwendung der Hautzustandsindikators 22 ist dabei in den Fig. 10 bis 12 gezeigt.

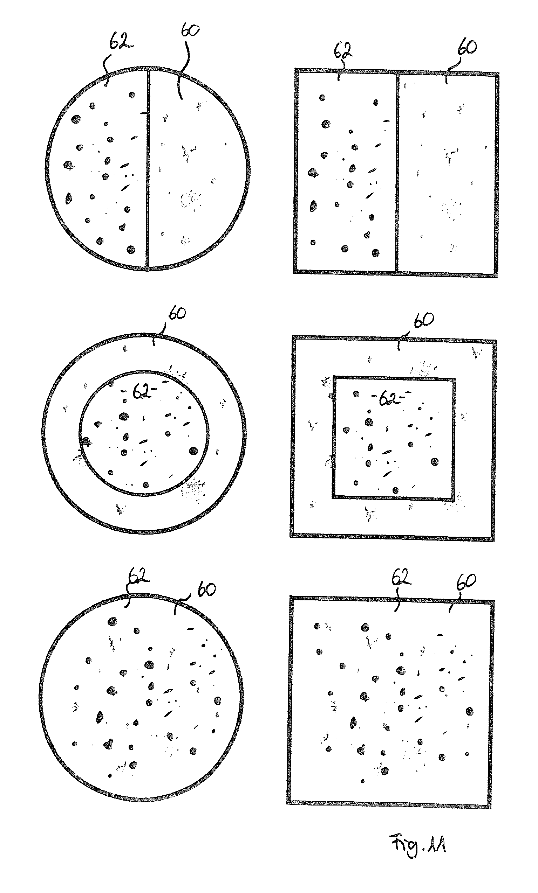

Der Hautzustandsindikator 22 wird beispielsweise auf die Wangenregion 12c aufgedrückt (Fig. 10). In Fig. 11 sind verschiedene Ausführungsformen des Indikatorbereiches dargestellt. Dabei können der Sebunbereich 62 und der Feuchtigkeitsbereich 60 räumlich getrennt sein, wie in den ersten vier Darstellungen gezeigt, oder die Bereiche 60, 62 sind überlagert, wie in den letzten beiden Darstellungen gezeigt.

Im vorliegenden Falle zeigt der Hautzustandsindikator eine geringe Menge an Feuchtigkeit und eine große Menge an Sebum an, d.h. es handelt sich hier um einen fett-trockenen Hautzustand.

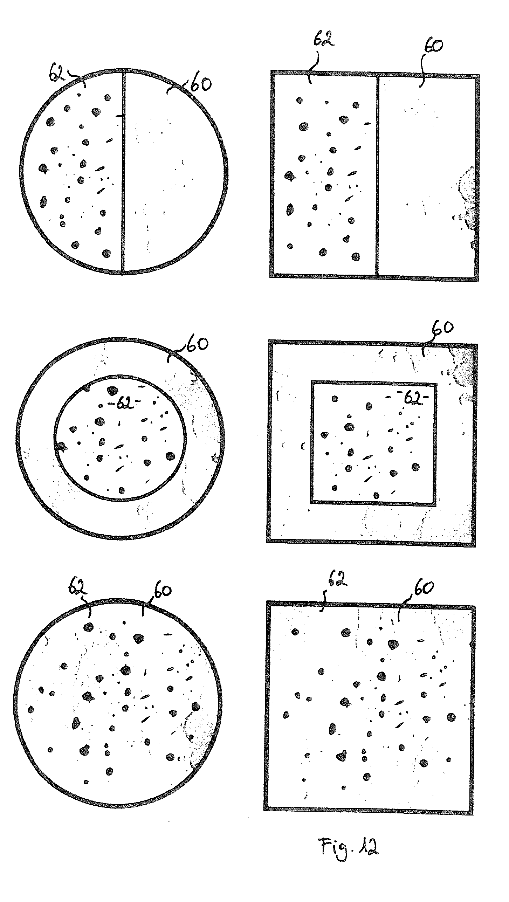

Im Falle der Fig. 12 wird deutlich mehr Feuchtigkeit angezeigt, so dass dieser Hautzustand als fett-feucht deklariert werden kann.

Die Fig. 13 und 14 zeigen Möglichkeiten, wie der beschriebene Hautzustandsindikator 22 zu einer Zeitschrift beigefügt werden kann. In Fig. 13 ist eine Möglichkeit dargestellt, bei der der Hautzustandsindikator 22 auf einer Broschüre 64 aufgebracht ist. Im Falle der Fig. 14 ist der Hautzustandsindikator 22 auf einem Flugblatt 66 aufgebracht. Zusätzlich zu dem Hautzustandsindikator 22 können auf der Broschüre 64 oder dem Flugblatt 66 Proben von Pflegeprodukten (nicht gezeigt) aufgebracht sein.

### Bezugszeichenliste

- 10: Gesichtsdarstellung
- 12: Hautzustandregion
- 12a: Stirnregion
- 12b: Kinnregion
- 12c: Wangenregion
- 22: Indikator, Hautzustandindikator
- 26: Träger
- 28: erste Schicht
- 30: Vergleichsmaßstab
- 32: zweite Schicht
- 34: Druckfarbe
- 36: Bindemittelmatrix
- 38: Licht
- 40: Pore
- 42: Additiv
- 44: Partikel
- 46: Additiv
- 48: wasserlöslicher Farbstoff
- 50: Lebensmittelfarbstoff
- 52: Farbfleck
- 54: erster Bereich
- 56: zweiter Bereich
- 58: dritter Bereich
- 60: Feuchtigkeitsbereich
- 62: Sebumbereich
- 64: Broschüre
- 66: Flugblatt

## Patentansprüche

1. Indikator (22) zur Ermittlung des Hautzustands, mit einem Träger (26) und mit einer auf dem Träger (26) aufgebrachten Schicht einer grafischen Druckfarbe (34), wobei die Druckfarbe (34) eine Bindemittelmatrix (36) aufweist und wobei die Bindemittelmatrix (36) wenigstens ein darin homogen dispergiertes Additiv (46) enthält, das zur irreversiblen Veränderung der optischen Eigenschaften der Bindemittelmatrix (36) bei Kontakt mir Hautsekret ausgebildet ist.

2. Indikator (22) nach Anspruch 1,
wobei das wenigstens eine Additiv (46) zur irreversiblen Veränderung der optischen Eigenschaften der Bindemittelmatrix (36) bei Kontakt mit Feuchtigkeit ausgebildet ist.

3. Indikator (22) nach Anspruch 1 oder 2,
wobei das wenigstens eine Additiv (46) bei Kontakt mit Feuchtigkeit zum irreversiblen Einfärben der Bindemittelmatrix (36) ausgebildet ist.

4. Indikator (22) nach einem der voranstehenden Ansprüche,
wobei das wenigstens eine Additiv (46) einen wasserlöslichen Farbstoff (48) aufweist, der Partikel (44) mit einer Größe von 1 bis 5 µm aufweist, wobei die Partikel (44) bei Reaktion mit Feuchtigkeit zum Erzeugen einer Farbgebung in der Bindemittelmatrix (36) zum Expandieren und Ausbluten in die Bindemittelmatrix (36) ausgebildet sind.

5. Indikator (22) nach Anspruch 4,
wobei der wasserlösliche Farbstoff (48) ein Lebensmittelfarbstoff (50) ist und/oder wobei die Bindemittelmatrix (36) wasserfrei und/oder ölfrei und/oder porös ist.

6. Indikator (22) nach einem der voranstehenden Ansprüche,
wobei die Bindemittelmatrix (36) ein zweites darin homogen dispergiertes Additiv (42) enthält, das zum irreversiblen Verändern der optischen Eigenschaften der Bindemittelmatrix (36) bei Kontakt mit Sebum ausgebildet ist.

7. Indikator (22) nach einem der voranstehenden Ansprüche,
wobei das zweite Additiv (42) bei Kontakt mit Sebum zum Verändern der Transmissionseigenschaften der Bindemittelmatrix (36) für sichtbares Licht (38) ausgebildet ist.

8. Indikator (22) nach Anspruch 6 oder 7,
wobei das zweite Additiv (42) wenigstens eine der folgenden Eigenschaften aufweist:
• es ist aus einer Gruppe ausgewählt, die SiO₂, Al₂O₃, Ca₃PO₄, Talkum und (Ca₅(PO₄)₃·OH) umfasst;
• es ist kristallin und liegt in einem Größenbereich von 1 bis 30 µm oder als Partikel in einem Größenbereich von unter 1 µm oder als Agglomerat in einem Größenbereich bis 300 µm vor.

9. Indikator (22) nach einem der voranstehenden Ansprüche,
wobei die Druckfarbe (34) eine wasserfreie erste Bindemittelmatrix (36) mit darin dispergiertem erstem Additiv (42) und eine ölfreie zweite Bindemittelmatrix (36) mit darin dispergiertem zweitem Additiv (42) aufweist.

10. Indikator (22) nach einem der voranstehenden Ansprüche,
wobei die Druckfarbe (34) eine Offsetdruckfarbe, eine Siebdruckfarbe oder eine Digitaldruckfarbe, insbesondere eine InkJet-Druckfarbe, ist und/oder wobei der Träger (26) aus Papier oder Kunststoff gebildet ist.

11. Indikator (22) nach einem der voranstehenden Ansprüche,
wobei der Träger (26) eine Farbgebung aufweist, unterschiedlich zu einer Farbgebung der Bindemittelmatrix (36) vor Kontakt des zweiten Additivs (42) mit Sebum ist und wobei die Farbgebung unterschiedlich zu der Farbgebung der Bindemittelmatrix (36) nach Kontakt des ersten Additivs (46) mit Feuchtigkeit ist und/oder wobei benachbart zu der Schicht der grafischen Druckfarbe (34) auf dem Träger (26) ein Vergleichsmaßstab (30) zur Anzeige des Hautzustands angeordnet ist.

12. Indikator (22) nach einem der Ansprüche 9 bis 11,
wobei der Träger (26) eine erste Schicht einer ersten Druckfarbe (34) mit einer wasserfreien Bindemittelmatrix (36) und einem darin dispergierten ersten Additiv (46) aufweist und wobei der Träger (26) eine zweite Schicht einer zweiten Druckfarbe (34) mit einer ölfreien Bindemittelmatrix (36) und einem darin dispergierten zweiten Additiv (42) aufweist.

13. Verfahren zur Herstellung eines Indikators (22) zur Anzeige des Hautzustands mit den Schritten
a) Dispergieren eines Additivs (42) in einer Bindemittelmatrix (36);
b) Vermischen der Bindemittelmatrix (36) mit einer grafischen Druckfarbe (34);
c) Bereitstellen eines Trägers (26);
d) Aufbringen von wenigstens einer Schicht (28) der Druckfarbe (34) mittels Drucktechnik auf den Träger (26).

14. Verfahren nach Anspruch 13,
wobei Schritt a) die Schritte
a1) Zermahlen eines wasserlöslichen Farbstoffes (48) zu einer Partikelgröße von 1 bis 5 µm und
a2) Bereitstellen einer wasserfreien und/oder ölfreien Bindemittelmatrix (36)
aufweist und/oder
wobei Schritt a) die Schritte
a3) Dispergieren eines zweiten Additivs (42) in einer zweiten Bindemittelmatrix (36);
a4) Vermischen der zweiten Bindemittelmatrix (36) mit einer grafischen zweiten Druckfarbe (34) und
a5) Aufbringen einer zweiten Schicht der zweiten Druckfarbe (34) mittels Drucktechnik auf den Träger (26)
aufweist.

15. Verfahren nach einem der Ansprüche 13 oder 14,
wobei das Aufbringen der ersten und/oder zweiten Druckfarbe (34) mittels Siebdruck, Tiefdruck oder Flexodruck durchgeführt wird.

16. Verfahren nach einem der Ansprüche 13 bis 15,
wobei die erste und/oder die zweite Schicht (28, 32) mit einer Trockenschichtdicke von 15 bis 20 µm aufgebracht wird und/oder wobei die erste und/oder die zweite Druckfarbe (34) in einer grafischen Gestaltung auf den Träger (26) aufgebracht werden und/oder wobei auf den Träger (26) benachbart zu der ersten und/oder zweiten Schicht (28, 32) ein Vergleichsmaßstab (30) aufgebracht wird.
